# NEW EUROPEAN PATENT SPECIFICATION

(11) **EP 0 632 015 B2**
(45) Date of publication and mention of the opposition decision: **04.10.2001**
(45) Mention of the grant of the patent: 27.05.1998
(21) Application number: 93201901.1
(22) Date of filing: 30.06.1993
(51) Int. Cl.: C07C 215/40, C07C 213/04, C07C 213/08

(54) **Reductive alkylation of amine to make tertiary amino polyol as precursor of fabric softening esters**
Reduktive Alkylierung von Amin zur Herstellung von tertiärem Aminopolyol als Ausgangsstoff für Wäsche weichmachende Ester
Alkylation réductive d'amine pour préparer un polyol d'amine tertiaire comme précurseur d'esters adoucissants pour le linge

(43) Date of publication of application: 04.01.1995
(73) Proprietor: THE PROCTER & GAMBLE COMPANY, Cincinnati, Ohio 45202 (US)
(72) Inventor: Hubesch, Bruno, B-3080 Tervueren (BE); Hardy, Frederick Edward, Newcastle Upon Tyne NE20 9ES (GB); Simpson, Anthony J., Cramlington, Northumberland NE23 8HB (GB)
(74) Representative: TER MEER STEINMEISTER & PARTNER GbR

(56) References cited:
- EP-A- 0 142 868
- EP-A- 0 359 956
- EP-A- 0 375 333
- FR-A- 2 356 627
- JP-A- 3 063 251
- US-A- 2 226 534
- US-A- 3 342 840
- US-A- 3 429 925
- US-A- 3 457 313
- US-A- 4 137 180
- US-A- 4 996 316
- DATABASE WPI Week 8430, Derwent Publications Ltd., London, GB; AN 84-186432 & RO-A-82 557 (STEK and ECON-)
- CHEMICAL ABSTRACTS, vol. 114, no. 15, 15 April 1991, Columbus, Ohio, US; abstract no. 141745f, J. VELISEK et al, "3-Chloro-1,2-propanediol-derived amino alcohol in protein hydrolyzates", page 634, column 1 &JOURNAL OF FOOD SCIENCE vol. 56, no. 1, 1991, CHICAGO US pages 136-138
- IN-A 148638 Chem. Abstr. 96:103639b (1982)
- J. March, 4th Ed. Seiten 898-899 (Wiley 1992)
- Il Farmaco Sci. 23(5), 441-7 (1968)
- J. Med Chem. 16(1), 23-7 (1973)
- Helv. Chim. Acta 73 97-105 (1990)
- Helv. Chim. Acta 70, 1786-90 (1987)
- J. Appl. Chem. USSR, 1738-9 (1990)
- Houben Weyl, Bd. XI/1, 1957, p. 24
- Chem. Abstracts, 115:70916, 1991, JP-A-03 063 251, Derwent Abst. 91-122581/17

## Description

The present invention describes an alternative reaction path for the making of quaternary ammonium esters for fabric softening.

The process starts from a chloroepoxide which is reacted with ammonia and water to form a primary amino polyol. The process continues as reductive alkylation of the primary and then secondary amine with aldehydes to provide an intermediate tertiary amino polyol. This first part of the process is followed by the traditional esterification and quaternisation steps known in the art for making quaternized ammonium fabric softening compounds.

### Background of the invention

The closest prior art to the present invention is US patent 4,137,180 which teaches the production of the same quaternized ammonium esters as the present invention and their use in fabric softeners.

The process to make these ammoniums has a different first part of the reaction route while the second part containing the esterification and quaternisation steps is identical. Hence the intermediate tertiary amino polyol of this prior art is provided by the alternative reaction of alkylation of a secondary amine with a chloroalkyl polyol by elimination of hydrochloric acid.

This first part of the known, prior art process to make the desired fabric softening ammoniums shows low selectivity, and uses for example dimethylamine as the secondary amine which is known to be highly poisonous and difficult to handle and has to be made e.g. from ammonia and methylchloride. Therefore the problem underlying the present invention is to provide an alternative process of high selectivity and using less difficult to handle and obtain raw materials while possibly still utilizing the easy parts of the known process.

Other patent disclosures include the US 3,342,840 disclosing the manufacture of a mono ester quaternary ammonium salt. US 4,696,771 describes a process for the preparation of amino alcohols or polyols by reaction of a secondary alcanol amine with a terminal epoxy compound for the manufacture of polyurethane.

US patent 4,540,821 and 4,654,376 both relate to the preparation of amino polyols by use of phenol, ammonia and ethylene oxide together with formaldehyde. Resulting products are used in textile softening compositions but are neither quaternized nor do they contain ester links.

### Summary of the invention

The invention relates to a process to provide a tertiary amino polyol of the formula (I) 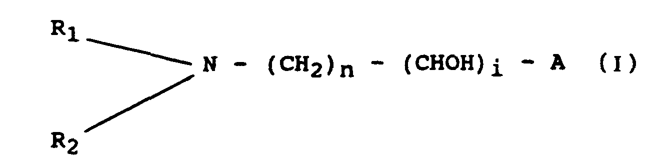 in which
R₁ and R₂ independently are (C₁ - C₈) - alkyl;
A is hydrogen or R₁;
n is 1,2,3,4,5 or 6;
i is 2,3,4,5 or 6
i + n is less than 9 if A is hydrogen and
i + n is less than 8 if A is not hydrogen.

This amino polyol is used as an intermediate compound for further esterification and quaternisation to produce a quaternized ammonium fabric softening compound of formula (II) by traditional process steps. 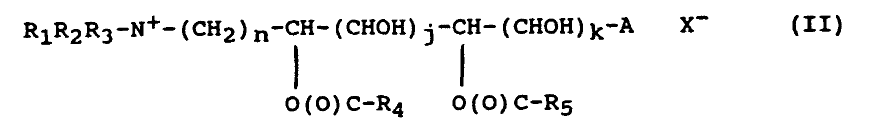
in which R₁, R₂, n, A are as in formula (I);
R3 is (C₁ - C₈) - alkyl or aryl;
j and k are independently 0,1,2,3 or 4 and j + k = i - 2 ;
R₄ and R₅ are independently (C₁₁-C₂₃) - alkyl or alkenyl.
X⁻ is a suitable counterion e.g. those disclosed in US,4,137,180. The process to make the intermediate tertiary amino polyol comprises the two steps which distinguish the claimed invention from the prior art.

The tertiary amino polyol is made by:
a) - reacting a halogen-CH₂-(C₂-C₈-alkyl)(1-3) epoxide or a halogen CH₂(C₂₋₆) polyalcohol with ammonia and water to form a primary amino polyol; and
b) - reductive alkylation of the resulting primary amino polyol with aliphatic (C₁ - C₈) - aldehydes to form the tertiary amino polyols of formula (I).

Preferred embodiments of the process of the present invention are in accordance with the dependent claims.

### Examples

In these examples the closest prior art (US 4,137,180) is compared with the present invention. For common conditions and reactions the details are therefore not given but can be referred to from prior art disclosures.

According to the present invention a α-chlorohydrin is reacted in an aqueous environment with ammonia according to the reaction path: 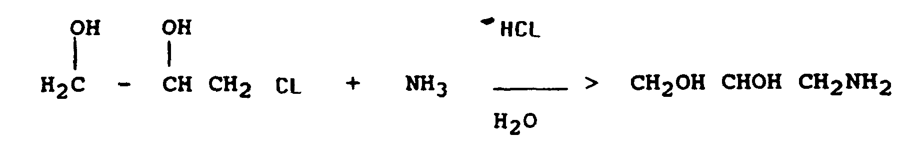 to result in a first intermediate mono amino polyol namely aminopropanediol while releasing hydrochloric acid.

The mono amino polyol is then reacted using formic acid or catalytic hydrogen e.g. with palladium with formaldehyde in a stoichiometric mole ratio of aminopropanediol to formaldehyde of 1:2. The reaction results after full conversion in dimethylaminopropanediol which is the starting amine to produce a quaternized ammonium compound as disclosed in the prior art. The reaction path to make dimethylaminopropanediol is : 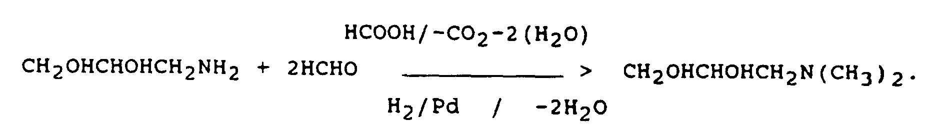 It has no alternative product as a result, i.e. the selectivity is 100%.

In the prior art US 4,137,180 the two process steps (to make the intermediate dimethylaminopropanediol) are given by the reaction paths 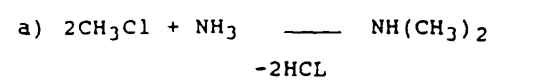 and

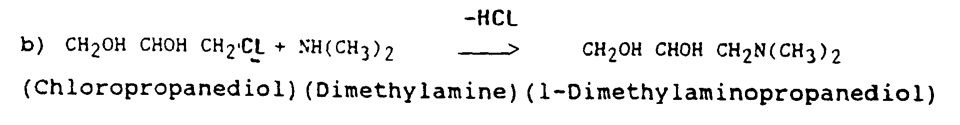

The second reaction has however the drawback that it may lead to stable side products namely quaternized dimethylammoniumdipropanediol, and by rearrangement to 2-dimethylaminopropanediol. These alternative reaction products of the reaction path b) reduces the process selectivity. Further it becomes necessary to eliminate the undesired process side products since they are not currently accepted as impurity in fabric softener compositions from a regulatory point of view in many countries.

In addition the resulting fabric softener compounds from these side products have no known softening benefit and hence may reduces overall softening efficiency.

## Claims

1. A process to provide a tertiary amino polyol of the formula (I) 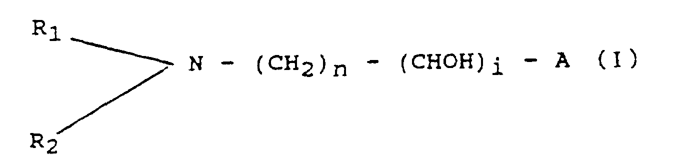 in which
R₁ and R₂ independently are (C₁ - C₈) - alkyl ;
A is hydrogen or R₁;
n is 1,2,3,4,5 or 6;
i is 2,3,4,5 or 6;
i + n is less than 9 if A is hydrogen; and
i + n is less than 8 if A is not hydrogen;
said process being **characterized in that** the tertiary amino polyol is made by 2 steps:
a) reacting a halogen-CH₂-(C₂-C₈-alkyl) (1-3) epoxide or a halogen -CH₂(C₂₋₆) poly-alcohol with ammonia and water to form a primary amino polyol; and
b) reductive alkylation of the resulting primary amino polyol with aliphatic (C₁-C₈)-aldehydes to form the tertiary amino polyols of formula (I).

2. A process according to claim 1 **characterized in that** i is 2; R₁, R₂, are independently (C₁ - C₂) - alkyl and A is a hydrogen.

3. A process according to any of the preceding claims **characterized in that** the epoxide is an epichlorohydrin or the poly-alcohol is a α-chlorohydrin.

4. A process according to any of the preceding claims **characterized in that** the reductive alkylation step b) uses catalytic hydrogen (palladium) or a chemical reducing agent, preferably formic acid.

5. A process according to any of the preceding claims **characterized in that** the aldehyde in step b) is formaldehyde.

6. A process according to claim 1, wherein said tertiary amino polyol is used as an intermediate compound for further esterification and quaternisation to produce a quaternized ammonium fabric softening compound of formula (II) 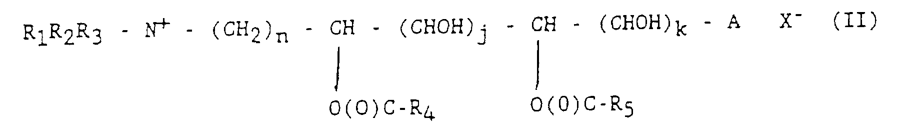
in which R₁, R₂, n, i, A are as in formula (I);
R3 is (C₁ - C₈) - alkyl or aryl;
j and k are independently 0,1,2,3 or 4 and j + k = i - 2 ;
R₄ and R₅ are independently (C₁₁-C₂₃) - alkyl or alkenyl; and
X⁻ is a suitable counterion.

7. A process according to claim 6, **characterised in that** R₁, R₂, R₃ are independently (C₁-C₂)-alkyl and A is hydrogen.

## Patentansprüche

1. Verfahren zur Herstellung eines tertiären Aminopolyols der Formel (I) 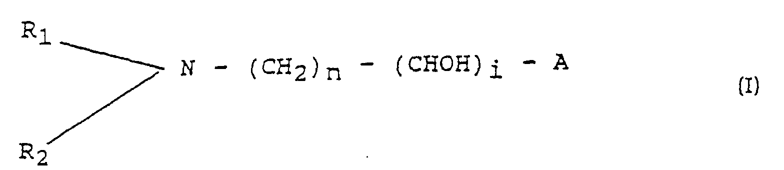 worin bedeuten:
R₁ und R₂ unabhängig voneinander (C₁-C₈)-Alkyl;
A Wasserstoff oder R₁;
n 1, 2, 3, 4, 5 oder 6;
i 2, 3, 4, 5 oder 6;
i + n weniger als 9, wenn A Wasserstoff ist; und
i + n weniger als 8, wenn A nicht Wasserstoff ist;
wobei das Verfahren **dadurch gekennzeichnet ist, daß** das tertiäre Aminopolyol in zwei Schritten hergestellt wird:
a) Umsetzen eines Halogen-CH₂-(C₂-C₈-alkyl) (1-3)-epoxids oder eines Halogen-CH₂-(C₂₋₆)-Polyalkohols mit Ammoniak und Wasser zur Bildung eines primären Aminopolyols; und
b) reduktive Alkylierung des resultierenden primären Aminopolyols mit aliphatischen (C₁-C₈)-Aldehyden zur Bildung des tertiären Aminopolyols der Formel (I).

2. Verfahren nach Anspruch 1, **dadurch gekennzeichnet, daß** i 2 ist; R₁ und R₂ unabhängig voneinander (C₁-C₂)-Alkyl sind und A Wasserstoff ist.

3. Verfahren nach mindestens einem der vorangehenden Ansprüche, **dadurch gekennzeichnet, daß** das Epoxid ein Epichlorhydrin ist oder der Polyalkohol ein α-Chlorhydrin ist.

4. Verfahren nach mindestens einem der vorangehenden Ansprüche, **dadurch gekennzeichnet, daß** beim reduktiven Alkylierungsschritt b) katalytischer Wasserstoff (Palladium) oder ein chemisches Reduktionsmittel, vorzugsweise Ameisensäure, verwendet wird.

5. Verfahren nach mindestens einem der vorangehenden Ansprüche, **dadurch gekennzeichnet, daß** der in Schritt b) verwendete Aldehyd Formaldeyd ist.

6. Verfahren nach Anspruch 1, wobei das tertiäre Aminopolyol als eine Zwischenverbindung zur weiteren Veresterung und Quaternisierung verwendet wird, um eine quaternisierte Ammonium-Wäscheweichmacherverbindung der Formel (II) herzustellen 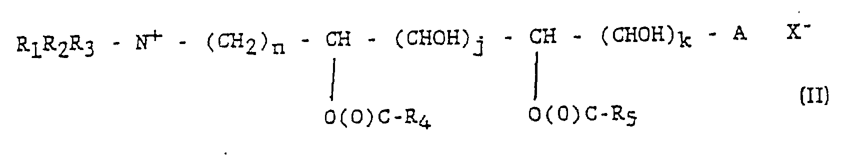
worin R₁, R₂, n, i, A wie in Formel (I) sind;
R₃ (C₁-C₈)-Alkyl oder Aryl ist;
j und k unabhängig voneinander 0, 1, 2, 3 oder 4 sind und j + k = i - 2;
R₄ und R₅ unabhängig voneinander (C₁₁-C₂₃)-Alkyl oder -Alkenyl sind; und X⁻ ein geeignetes Gegenion ist.

7. Verfahren nach Anspruch 6, **dadurch gekennzeichnet, daß** R₁, R₂ und R₃ unabhängig voneinander (C₁-C₂)-Alkyl sind und A Wasserstoff ist.

## Revendications

1. Procédé pour préparer un aminopolyol tertiaire de formule (I) 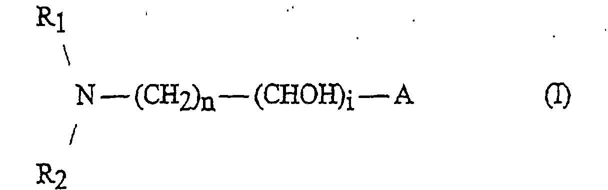 dans laquelle
R₁ et R₂ sont indépendamment des groupes alkyle en C₁-C₈;
A est un atome d'hydrogène ou R₁;
n vaut 1, 2, 3, 4, 5 ou 6 ;
i vaut 2, 3, 4, 5 ou 6 ;
i + n vaut moins de 9 si A est un atome d'hydrogène ; et
i + n vaut moins de 8 si A n'est pas un atome d'hydrogène ;
ledit procédé étant **caractérisé en ce que** l'aminopolyol tertiaire est préparé en 2 étapes :
a) réaction d'un halogène-CH₂-(alkyl en C₂-C₈)-époxyde-1,3 ou d'un halogène-CH₂-(polyol en C₂-C₆) avec de l'ammoniac et de l'eau pour former un aminopolyol primaire; et
b) alkylation réductrice de l'aminopolyol primaire résultant avec des aldéhydes aliphatiques en C₁-C₈ pour former les aminopolyols tertiaires de formule (I).

2. Procédé selon la revendication 1, **caractérisé en ce que** i est égal à 2 ; R₁, R₂ sont indépendamment des groupes alkyle en C₁-C₂ et A est un atome d'hydrogène.

3. Procédé selon l'une quelconque des revendications précédentes, **caractérisé en ce que** l'époxyde est une épichlorhydrine ou le polyol est une α-chlorhydrine.

4. Procédé selon l'une quelconque des revendications précédentes, **caractérisé en ce que** l'étape d'alkylation réductrice b) emploie de l'hydrogène catalytique (palladium) ou un agent réducteur chimique, de préférence l'acide formique.

5. Procédé selon l'une quelconque des revendications précédentes, **caractérisé en ce que** l'aldéhyde dans l'étape b) est le formaldéhyde.

6. Procédé selon la revendication 1, dans lequel ledit aminopolyol tertiaire est utilisé comme composé intermédiaire destiné ensuite à une estérification et à une quaternisation pour produire un composé adoucissant textile de type ammonium quaternisé de formule (II) 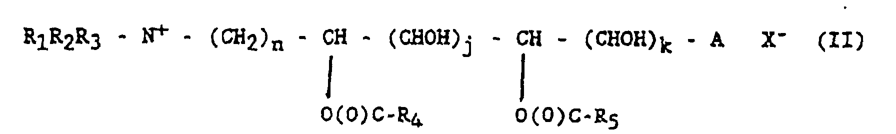
dans laquelle R₁, R₂, n, i, A sont comme dans la formule (I) ;
R₃ est un groupe alkyle ou aryle en C₁-C₈ ;
j et k sont indépendamment égaux à 0, 1, 2, 3 ou 4, et j + k = i - 2 ;
R₄ et R₅ sont indépendamment des groupes alkyle ou alcényle en C₁₁-C₂₃ ; et
X- est un contre-ion convenable.

7. Procédé selon la revendication 6, **caractérisé en ce que** R₁, R₂, R₃ sont indépendamment un groupe alkyle en C₁-C₂ et A est un atome d'hydrogène.
